# EUROPEAN PATENT SPECIFICATION

(11) **EP 2 586 390 B1**
(45) Date of publication and mention of the grant of the patent: **29.08.2018**
(21) Application number: 12199356.2
(22) Date of filing: 25.02.2004
(51) Int. Cl.: A61B 17/50, A61H 7/00

(54) **Device for mechanical treatment of early stage acne**
Vorrichtung zur mechanischen Behandlung von Akne im Frühstadium
Appareil de traitement mécanique de l'acné précoce

(43) Date of publication of application: 01.05.2013
(62) Divisional of application: 04785834.5
(73) Proprietor: L'OREAL, S.A., 75008 Paris (FR)
(72) Inventor: Pilcher, Kenneth, Seattle, WA Washington 98107 (US); Giuliani, David, Mercer Island, WA Washington 98040 (US); Meginniss, Stephen, Seattle, WA Washington 98119 (US)
(74) Representative: Nony

(56) References cited:
- EP-A1- 0 978 269
- WO-A1-03/096860
- WO-A1-03/096961
- CH-A5- 616 075
- DE-C1- 4 223 516
- JP-A- 2000 139 567
- US-A- 2 076 410
- US-A- 5 687 442

## Description

### Technical Field

This invention relates generally to treatment of early stage acne, and more particularly concerns mechanical and/or acoustic devices for applying energy to the skin in the vicinity of the early stage acne lesion, i.e. the sebaceous plug.

### Background of the Invention

Common acne, known more specifically as acne vulgaris, is generally regarded to be the most treated skin condition in the United States. Prompt and appropriate treatment of acne, particularly in its early stages, is important for both resolving the early stage condition and preventing more severe acne conditions, which have possible permanent effects, including the possibility of severe scarring. While acne can occur in men and women of all ages, it typically occurs in adolescents and young adults.

The earliest evidence of acne is the formation of a sebaceous plug. The sebaceous plug, which is formed in the individual skin pores (follicles), is typically not visible to the unassisted eye, but can be seen under a microscope or other optical lens device. It is formed when a combination of corneocytes and sebum, which are both natural components of the skin, block the pore opening, and specific colonies of bacteria within the skin pore then expand in numbers. The plug of cells and sebum may adhere to the wall of the skin pore, leading to material aggregation in the pore, and subsequent widening of the pore. This situation may in turn result in the further accumulation of sebum and other cellular material, and the eventual possible rupture of the follicular wall, followed by an inflammatory response and the subsequent formation of inflamed papules and inflamed pustules, typically referred to as pimples.

Existing systemic treatments of acne include oral antibiotics, retinoids and hormonal treatments. Each of these treatments, while effective to various extents, has its own significant side effects and disadvantages. For instance, oral antibiotic treatment reduces the number of bacteria in the skin pores, but does not decrease the rate of sebum secretions or the actual number of the sebaceous plugs formed. Disadvantages of the various treatments include various undesirable skin reactions, including skin dryness, fluid loss and possible hair loss. Typically, all such treatments irritate the skin to some extent.

### Prior Art

Prior art concerning localized treatment for acne can be classified generally as "mechanical" or "chemical."

Mechanical methods include vacuum devices, mechanized scrub brushes and manual loop-like instruments, such as shown in U.S. Patents No. 5,624,416 and 4,175,551. Use of these devices is typically site-specific and usually requires a specific technique, making them difficult to use. Methods that use heat generated by electrical resistance or ultrasound are also known, such as shown in U.S. Patent No. 6,245,093. Still other methods claim to be able to kill target micro-organisms, including those that cause acne, using selected frequencies of electrical current, such as shown in U.S. Patent No. 5,891,182.

CH 616 075 discloses a water operated massaging device, comprising a mechanism for reciprocally rotating a brush configured for contacting the skin. Water is sprayed on the skin as the device is operated.

WO03/096860 discloses an apparatus for treating a person's skin comprising a motor-driven skin treatment device, preferably formed by a brush, which device can be driven into rotation and can additionally be driven into vibration.

In the beauty/skin care industry, the use of microabrasion is also a popular treatment for "rejuvenating" skin. However, this technique of removing some layers of the cornified skin layer by abrasive materials can cause intense irritation.

Chemical methods for acne, including topical and systemic treatments and their possible side effects, are listed in Tables I and II below, respectively.

**Table I. Common Topical Acne Treatments**

| **Treatment** | **Possible Side Effect** |
|---|---|
| Soaps and detergents to remove sebum from skin surface | Drying of skin surface (xerosis) if used in excess |
| Astringents and short chain alcohols to remove oily materials and water in upper epidermal areas | Drying of the skin surface and breakdown of the skin's barrier function, and eventual microbial entrance into the body |
| Antibacterial agents (e.g. benzoyl peroxide, salicylic acid), that can destroy bacteria when the agent is in immediate contact with the microorganisms | <5% penetrates into the pores, with the rest possibly interacting with corneocytes causing irritation and erythmea (red skin), and contact dermatitis |

**Table II. Common Systemic Acne Treatments**

| **Treatment** | **Possible Side Effect** |
|---|---|
| Oral antibiotics | Photosensitivity, gastrointestinal problems, and bacterial antibiotic resistance |
| Hormonal manipulations to control sebaceous gland size and secretion rate by regulating androgens and estrogens | Increased risk of thromboembolism, feminization in men, and other undesirable effects. |
| Retinoids, which likely change the cohesiveness of follicular epithelial cells | Teratogenic, and other strong negative side effects |

With the present apparatus, conditions that lead to early stage acne are prevented and early stage acne is effectively treated by maintaining or restoring the pore openings to an open state, to allow continuing exudation from the sebaceous gland, to encourage maintaining an aerobic state within the pore, and to prevent the development of more severe acne conditions, without the inconvenience, side effects and other limitations present in existing treatments.

### Disclosure of the Invention

Accordingly, the invention is an apparatus according to claim 1.

### Brief Description of the Drawings

Figure 1 shows a cross-sectional view of a typical skin pore.
Figure 2 is a cross-sectional view of a comedone-plugged acroinfundibulum.
Figure 3 is a cross-sectional view of the comedone-plugged acroinfundibulum, taken along lines 3-3 in Figure 2.
Figure 4 shows strain characteristics for skin for differing degrees of applied stress.
Figures 5A and 5B show the relative displacement of a single tuft of bristles moving against the skin.
Figure 6 is an exploded view of one embodiment of a mechanical acne treatment device useful for understanding the present invention.
Figure 7 is a schematic view of a fixed contact element part of the apparatus of Figure 6.
Figure 8 is a schematic view of a movable contact element part of the apparatus of Figure 6.
Figures 9A, 9B, 9C and 9D show the effect on a typical sebaceous plug positioned within a skin pore subjected to the action of the apparatus of Figure 6.
Figure 10 is a schematic view of a variation of the mechanical device of Figure 6.
Figure 11 is a schematic view of a fixed contact element part of the apparatus of Figure 10.
Figure 12 is a schematic view of a movable contact element part of the apparatus of Figure 10.
Figures 13A, 13B, 13C and 13D show the effect on a sebaceous plug positioned in a skin pore subjected to the action of the apparatus of Figures 10-12.
Figure 14 shows a variation of the mechanical device shown in Figure 7, wherein a compliant material is applied to the contact element surface.
Figure 15 is a diagram showing an embodiment of the present invention with alternately linearly movable rows of bristle tufts, surrounded by a circular row of fixed bristle tufts.
Figure 16 is a top view of the device shown in Figure 15.
Figure 17 is a diagram showing a variation of the mechanical device shown in Figure 15, in which one set of bristle tufts is fixed, and the other set moves linearly.
Figure 18 is a top view of the device shown in Figure 17.
Figure 19 is a diagram showing a further alternate embodiment of the present invention with alternately rotationally movable sets of bristle tufts.
Figure 20 is a top view of the mechanical device shown in Figure 19.
Figure 21 is a diagram showing a variation of the mechanical device shown in Figure 16 with a single set (two rows) of rotationally moving bristle tufts.
Figure 22 is a top view of the mechanical device shown in Figure 21.
Figure 23 is a schematic block diagram showing the control means for controlling the amplitude of motion as a function of pressure applied to the skin.
Figure 24 is a schematic representation of the human face showing the various regions of the face that differ in the degree of sebum secretion and incidence of acne lesions.
Figure 25 is a block diagram of a timer structure useful with the present invention.

### Best Mode for Carrying Out the Invention

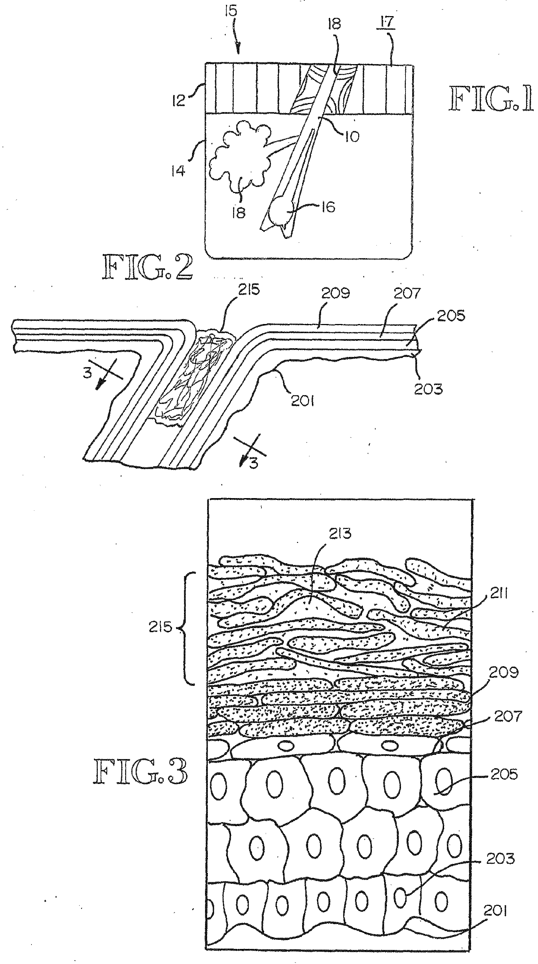

Figure 1 is a representation of a typical skin pore, including the epidermis and dermis layers of the skin. The skin pore 10, also referred to as a follicle, includes a normal hair 16 and an associated sebaceous gland 18. The sebaceous gland 18 normally produces sebum lipids. The production of sebum, however, is typically not sufficient alone to result in acne. Further, acne lesions do not occur if the sebum lipids are free to reach the surface of the skin. However, when the skin pore or follicle becomes blocked, such as by an overproduction of corneocytes, or inadequate shedding of the corneocytes (as shown in Figure 2), the balance of the skin system is upset. The blocked follicles lead to the formation of closed, but non-inflamed, sebaceous plugs. The sebaceous plug (microcomedone) 215 shown in vertical cross-section in Figure 2 and in horizontal cross-section in Figure 3 forms in the acroinfundibulum portion of the follicle, which is the upper portion of the follicle.

Following the initial formation of the sebaceous plug, if the pH and oxygen tension are within certain ranges below the closed sebaceous plug, the number of Propionibacteria acnes bacteria expands, leading to a pathogenic condition. This leads further to a sequence of actions and reactions within the follicle, including damage to the follicular wall, comprising skin layers 201, 203, 205, 207 and 209, and extrusion of accumulated materials into the dermis portion of the skin, resulting in an inflammatory response which leads to skin lesions and pustules.

In the present invention, the focus is on maintaining the acroinfundibulum portion of the follicle in an open state, which eliminates the environment in which the acne bacteria can thrive within the follicle, and encourages establishing an aerobic state within the follicle, while at the same time minimizing the amount of sebum that can accumulate within the infundibulum portion of the follicle.

The basic approach of the present invention is to reopen the individual pores that may have been blocked by the plug of corneocytes 211 and sebum lipids 213 (Figure 3). It is based on the discovery that application of differential motion locally to the pore opening will open a blocked pore. The opening of the pore is due to the fact that the blocking materials within the follicles have different physical properties than the wall of the infundibulum and the surrounding skin. With the present invention, the skin area is deformed slightly and then released to a relaxed position and then deformed slightly in the opposite direction and then again released to a relaxed position, at a specified frequency, which results in the plugs being loosened from their position in the skin pores. The loosened plugs can then be readily removed, such as by wiping or washing, permitting thereafter normal skin secretion of lipids, and consequently avoiding the consequences of more fully developed acne.

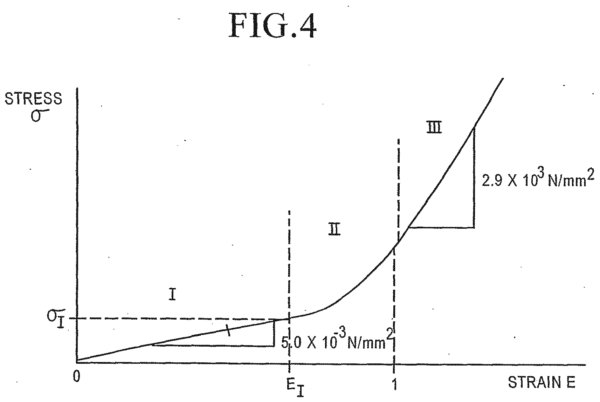

Figure 4 shows three regions of the skin's elastic modulus, i.e. the amount force (stress) required to deform the skin a given degree (strain). This curve is the result of the unique mechanical organization of the skin. This mechanical organization can be thought of as large numbers of loose collagen fibers connected together at randomly distributed nodal points. The mechanical behavior of such a system is very similar to that of a woven material such as a nylon stocking. As the material is stretched, the fibers are first straightened out and become oriented in the direction of the stress (shown in Figure 4 region I). A relatively small amount of stress is required to produce this level of strain, with a modulus of elasticity typically 5 x 10⁻³ N/mm². Skin is elastic over this range.

Generally at the end of region I and slightly into region II, the elasticity of the skin substantially decreases and the skin becomes taut. In region II, some fibers become fully aligned in the direction of the stress and then carry stress directly. Further deformation will result in ever-increasing numbers of collagen fibers being recruited to support the stress. The modulus of elasticity, or stiffness, of the skin increases rapidly as this process continues until it matches the stiffness of the collagen fibers themselves (region III). The modulus of elasticity in this region is typically 3 x 10³ N/mm².

In the present invention, the desired differential motion applied to the skin should be of high enough amplitude to create pore opening forces, but low enough to minimize stretching of collagen fibers in the skin. Deformation should be limited to the area of region I and the low strain area of region II of Figure 4, where the collagen fibers are not significantly stretched. To that end, the mechanical characteristics of the portions of the invention that contact the skin, called the contacting elements, and the amplitude of the moving contact elements must be such that the degree of stress on the skin does not exceed some value σ_{I} thus keeping the amount of strain in the skin below the value E_{I} of Figure 4.

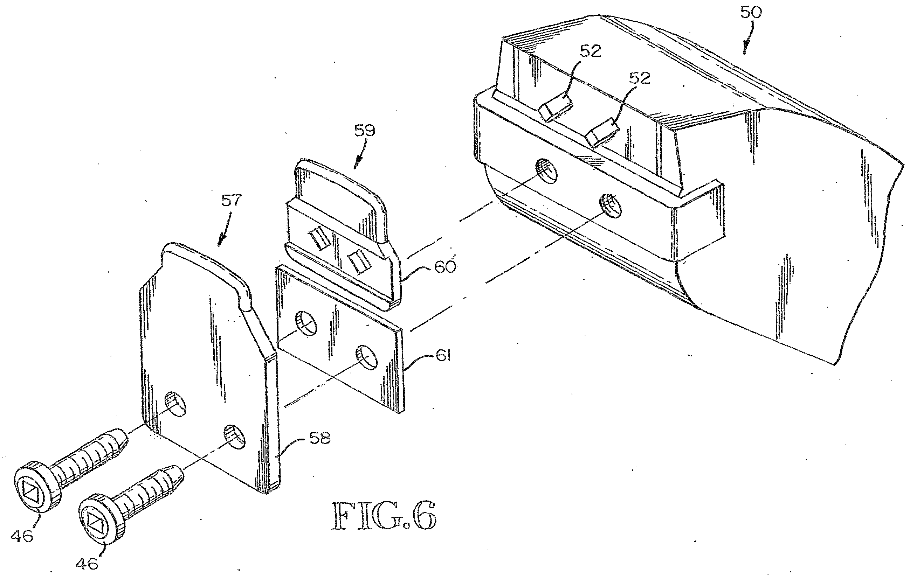

A first embodiment useful for understanding the present invention is shown generally in Figure 6, wherein movable skin contacting elements are located in the same plane as stationary skin contacting elements, and the bi-directional differential action is shear, i.e. the two elements move in parallel to each other along their length. The arrangement shown includes two skin contact elements 57 and 59 and a backing/spacer plate 61.

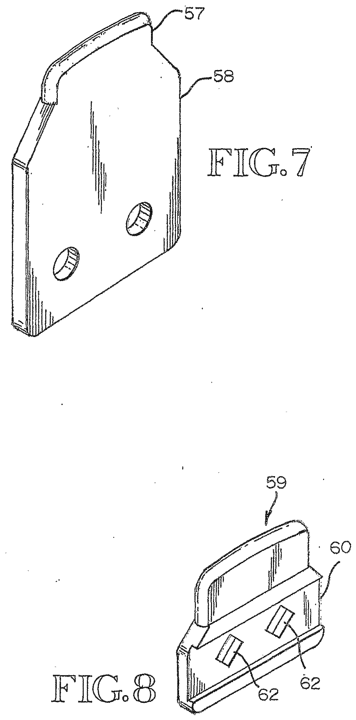

The movable and non-movable skin contacting elements are basically identical. In the embodiment shown, the individual contact elements 57 and 59 are each mounted on mounting plates. The fixed contact element 57 is mounted on mounting plate 58, while the oscillating contact element 59 is mounted on mounting plate 60. The contact elements are narrow and somewhat elongated and are shown in detail in Figures 7 and 8. In the embodiment shown, the dimensions of the contact elements are typically 20.3 mm (0.800 inches) long and 2.30 mm (0.09 inches) wide. The edges of each contact element are beveled; giving it a somewhat rounded end face, while the respective ends are also rounded, as shown in the drawings.

The mounting plate 58 for the fixed contact element is approximately 30.0 mm (1.18 inches) wide. The upper corners of mounting plate 58 are both cut off, at a 36° angle. The height of the mounting plate 58 is 36 mm (1.4 inches). Figure 8 shows the combination of contact element 59 and mounting plate 60 for the oscillating contact element. Mounting plate 60 is, in the embodiment shown, substantially rectangular, with a length of 25 mm (1.0 inches) and a height of 12.7 mm (0.5 inches). The upper corners of mounting plate 60 are cut off, *i.e*, beveled, at an angle of 45°, while the lower corners are rounded.

Oscillating contact element 59 is mounted on mounting plate 60 on the upper edge thereof. The upper edge of contact element 59 is approximately 7.57 mm (0.298 inches) above the upper edge of mounting plate 60. Mounting plate 60 includes two drive openings 62-62 therethrough, so that the mounting plate 60 and contact element 59 can be moved back and forth by a driver mechanism discussed below. In the embodiment shown, the drive holes 62 are approximately square, 3.91 mm (0.154 inches) on each side.

The oscillating contact element 59 on its mounting plate 60 and the fixed contact element 57 on its mounting plate 58 are then positioned immediately adjacent to each other, as shown in Figure 6. The two contact elements 57 and 59 are thus substantially in registry. The two assemblies are held together and attached to the driver mechanism 50 by two connecting screws 46-46. Hence the contact elements may be removed and replaced. The oscillating contact element assembly is movable by means of the driver that moves it reciprocally (back and forth), such that the mounting plate 60 moves parallel to mounting plate 58, and contact element 59 moves reciprocally in parallel with contact element 57.

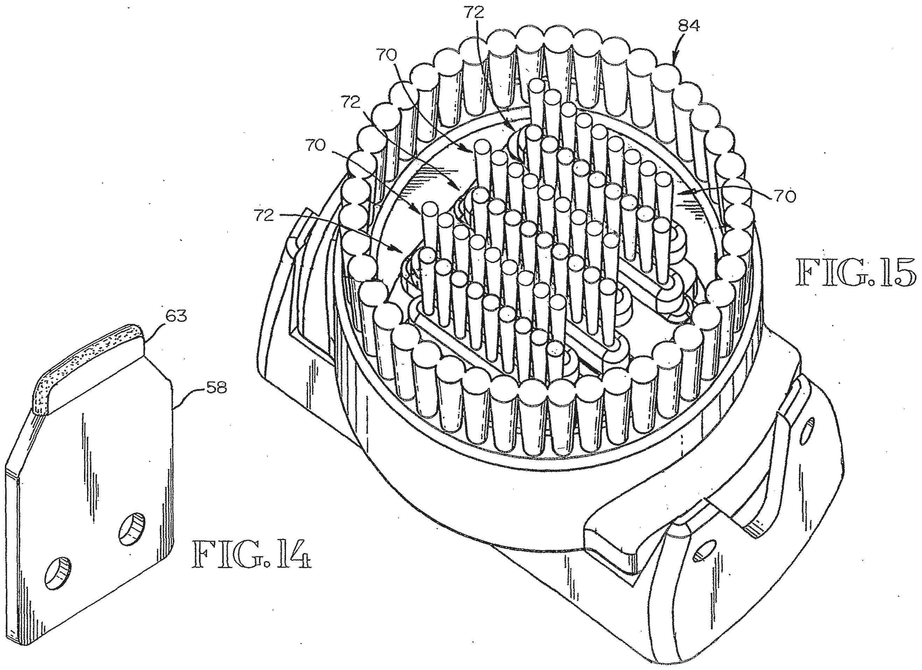

The drive assembly shown generally at 50 includes two drive buttons 52-52 that move reciprocally a selected distance. These drive buttons extend into drive openings 62-62 on the oscillating mounting plate. The oscillating contact element in the embodiment shown has a frequency within the range of 20 Hz to 1 kHz, with a preferred value range of 80-200 Hz. As indicated above, the action of the drive assembly moves the mounting plate 60 parallel with mounting plate 58, so that the oscillating contact element 59 moves parallel to the length of the adjacent fixed contact element 57. In the embodiment shown, the contact element and the mounting plates are made from stainless steel, although the contact elements could also be coated with a compliant material or be composed entirely of compliant material, such as shown at 63 in Figure 14, or the contact elements could be replaced by bristle brush tufts or the like.

In the embodiment shown, a center-to-center distance of approximately 3.18 mm (0.125 inches) results in a separation between 2.29 mm (0.09 inches) wide contact elements 57 and 59 of approximately 0.889 mm (0.035 inches). Contact element 59 moves reciprocally over a total distance in a range of 0.508 mm (0.02 inches) to 2.03 mm (0.08 inches), with a preferred value of approximately 1.02 (0.040 inches) (+/- 0.508 mm (0.020 inches) from its neutral position to its peak position) along contact element 57. The surface finish of the contact elements 57 and 59 is such that the skin primarily moves in contact with the contact elements. A surface roughness range of 0.127 µm (5 microinches) to 0.508 µm (20 microinches) is effective, with a preferred value of 0.254 µm (10 microinches). The surfaces must be sufficiently rough that the motion of the contact elements is transferred to the skin with minimal or no slippage. If the surface is too smooth, the skin could be abraded. The contact element could be an elastomer or a closed cell foam. It could be a knobby surface or even fingers.

In operation of the embodiment of Figure 6, the edge faces of the contact elements will be positioned lightly against the skin surface and the device activated by a switch. The contact element 59 begins to oscillate. The device is then moved at a slow rate across the skin surface, for instance two centimeters per second. The device operates with a ratio of peak amplitude to the space (distance) between adjacent skin contacting elements of typically 0.57. With that action, shear forces are applied to the skin, with sufficient amplitude to slightly distort the skin and force open the pores, but low enough to minimize any skin stretching.

At the above-noted frequency range, with a minimum of 20 Hz, each pore opening is deformed approximately 10 times per second. At higher frequencies, the number of deformations per second would be proportionately greater. Alternating shear stress in the tissue surrounding the infundibulum is produced, with the adhesion between the sebaceous plug and the infundibular wall being weakened or significantly reduced, so that the plug is essentially loosened in the pore.

While the embodiment of Figure 6 shows one fixed contact element and one movable contact element, it should be recognized that a plurality of fixed contact elements and oscillating contact elements could be used to provide a wider coverage for the device. In the case of a plurality of contact elements, the movable multiple contact element(s) are interdigitated with the fixed contact element(s) and are driven in a ganged manner.

In addition, both contact elements can be driven, preferably in equal and opposite directions of motion with respect to each other. A peak amplitude of 0.508 mm (0.02 inches) for each of two moving elements would result in a peak amplitude of relative motion of 1.02 mm (0.04 inch).

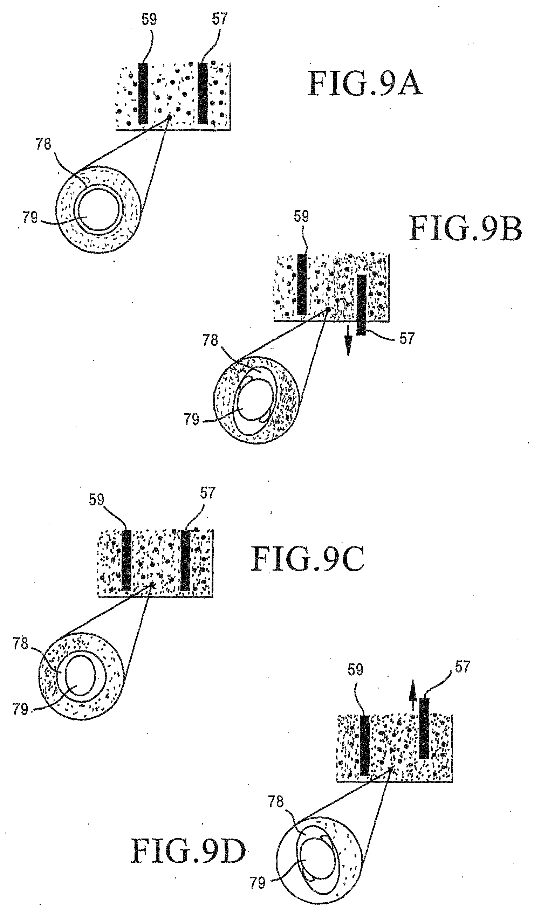

Figures 9A-9D show the action on the skin and a sebaceous plug with the shear embodiment of Figures 6-8. Figure 9A shows a pore 78 blocked by a sebaceous plug 79 therein. The contact elements are in a neutral position. The movable contact element will then be moved in one direction, in parallel with the fixed contact element, which distorts the sebaceous plug (Figure 9B). The movable contact element and mounting plate combination is then reversed and returns to the neutral position. This is shown in Figure 9C. The movable contact element continues in the opposite direction, which deforms the sebaceous plug in the opposite direction (Figure 9D). This is accomplished at the specified frequency. While generally this "double" motion is preferred, it is possible to move the movable contact in one direction only relative to the neutral/rest position. Continued repetitive action dislodges or releases the sebaceous plug from the pore walls. The device is slowly moved across the surface of the skin, producing the above results over an entire skin area.

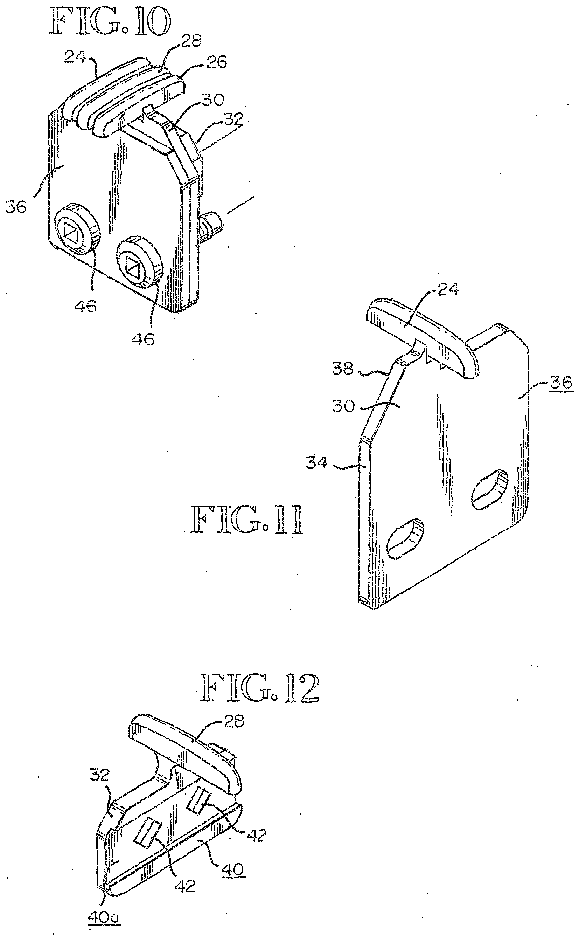

An alternative mechanical arrangement is shown in Figures 10-12. It includes two fixed skin contact elements 24 and 26 and an intermediate oscillating contact element 28. The configuration of the elements in the tension/compression arrangement is similar to that of the elements of the embodiment of Figures 6-8, although the "shear" action of the embodiment of Figures 6-8 is generally preferred. In both embodiments, the differential strain on the skin produced by the mechanical action is sufficient to result in a breaking away of the plug from the skin, due to the difference in elasticity between the plug material and the skin.

In the embodiment of Figures 10-12, the individual contact elements 24, 26 and 28 are each mounted on mounting plates. The fixed contact elements 24, 26 are mounted on mounting plates 30-30 (Figure 11), while the oscillating contact element 28 is mounted on mounting plate 32 (Figure 12). The contact elements are narrow and somewhat elongated and are shown in detail in Figures 11 and 12. In the embodiment shown, the contact elements are typically approximately 20.3 mm (0.800 inches) long and approximately 2.29 mm (0.090 inches) thick. The edges of each contact element are beveled; giving each contact element a somewhat rounded end face, while the respective ends are also rounded, as shown in the drawings. The mounting plate 30 for the fixed contact element is approximately 30.0 mm (1.18 inches) wide. The upper corners of mounting plate 30 are both cut off, at a 36° angle. The height of the contact element is somewhat less (31.8 mm (1.25 inches)) on one side of the contact element relative to the other side (36 mm (1.4 inches)).

In the embodiment shown, the fixed contact elements 24, 26 are mounted cross-wise (perpendicular) to the mounting plate, approximately 12.7 mm (0.5 inches) from one edge 34 thereof. The fixed contact elements are offset laterally, such that they extend approximately 8.03 mm (0.316 inches) from one surface 36 of mounting plate 30, and approximately 10.3 mm (0.406 inches) from the opposing surface 38.

Figure 12 shows the combination of contact element 28 and mounting plate 32 for the oscillating contact element. Mounting plate 32 is, in the embodiment shown, substantially rectangular, with a length of 25.4 mm (1.0 inches) and a height of 12.7 mm (0.5 inches). The upper corners of mounting plate 32 are cut off, i.e. beveled, at an angle of 45°, while the lower corners are rounded.

Oscillating contact element 28 is mounted perpendicularly to mounting plate 32 on the upper edge thereof. The upper edge of contact element 28 is approximately 7.57 mm (0.298 inches) above the upper edge of mounting plate 32 and is offset, so that it extends approximately 12.0 mm (0.472 inches) from surface 40 of mounting plate 32. Mounting plate 32 includes two drive holes 42-42 therethrough; so that the mounting plate 32 and contact element 28 can be moved back and forth by a driver mechanism discussed below. In the embodiment shown, the drive holes 42 are approximately square, 3.91 mm (0.154 inches) on each side.

The oscillating contact element 28 on its mounting plate 32 and the fixed contact elements 24 and 26 on their mounting plates 30 are then positioned immediately adjacent to each other, with the two fixed contact element assemblies being back-to-back, but reversed, as shown in Figure 10. The three contact elements 24, 26 and 28 are thus substantially in registry. The three assemblies are held together and attached to the driver by two connecting screws 46-46. The oscillating contact element assembly is movable by means of a driver that moves it back and forth, such that the mounting plate 32 moves parallel to mounting plates 30, and contact element 28 moves alternately toward and away from contact elements 24 and 26.

A drive assembly similar to that shown at 50 in Figure 6 includes two drive buttons which move back and forth a selected distance. These drive buttons connect to the drive holes 42-42 on the oscillating mounting plate 32. The oscillating contact element in the embodiment shown moves at a frequency within the range of 20 Hz to 1 kHz, with a preferred range of 80-200 Hz. As indicated above, the action of the drive assembly moves the mounting plate 32 parallel with mounting plates 30, so that the oscillating contact element 28 moves toward (or away from) one adjacent fixed contact element 24 and away from (toward) the other adjacent fixed contact element 26.

In the embodiment shown, contact elements 24 and 26 are separated by a center-to-center distance of approximately 7.11 mm (0.280 inches) and contact element 28 moves reciprocally over a peak-to-peak distance of approximately 3.81 mm (0.150 inches) between contact elements 24 and 26. Movement between a neutral/rest position and a peak distance (one direction) and back to neutral is also possible. In operation of the embodiment of Figures 10-12, the edge faces of the contact elements will be positioned lightly against the skin surface and the device activated by a switch. The contact element 28 begins to oscillate. The device is then moved at a slow rate across the skin surface, for instance two centimeters per second. With that action, shear forces of tension and compression are applied to the skin, with sufficient amplitude to slightly force open the pores, but low enough to minimize any skin stretching or deformation. In this embodiment, the peak amplitude of motion is approximately 39% of the spacing between adjacent contact elements.

At the above-noted frequency range, with a minimum of 20 Hz, each pore opening is deformed approximately 10 times per second. At higher frequencies, the number of deformations per second would be proportionately greater. Alternating tension and compression stress in the tissue surrounding the infundibulum results, with the adhesion between the sebaceous plug and the infundibular wall being weakened or significantly reduced, so that the plug becomes essentially loose in the pore.

While the embodiment of Figures 10-12 shows two fixed contact elements and one movable contact element, it should be recognized that only one fixed contact element could be used, or a plurality of fixed contact elements and oscillating contact elements can be used to provide a slightly wider coverage. In the case of a plurality of contact elements, the moving multiple contact element(s) are interdigitated with the fixed contact element(s), and are driven in a ganged manner.

In addition, both contact elements can be driven, preferably in equal and opposite directions of motion with respect to each other. A peak amplitude of 0.508 mm (0.02 inches) for each of two moving elements would result in a peak amplitude of relative motion of 1.02 mm (0.04 inches).

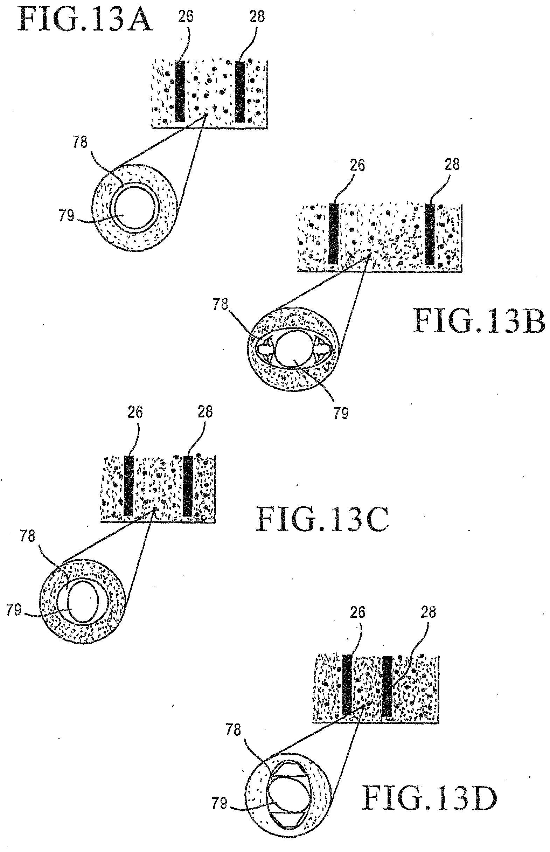

Figures 13A-13D show action on a pore with a sebaceous plug with the tension/compression arrangement of Figures 10-12.

Figure 13A shows a pore 78 blocked by a sebaceous plug 79 therein. The contact elements are in a neutral position. The movable contact element will then be moved in one direction, perpendicularly away from the fixed element, which deforms the sebaceous plug and causes deformation of the pore in one direction (Figure 13B). The motion is then reversed and returns to the neutral position, relaxing the force between the sebaceous plug and the acroinfundibulum, as shown in Figure 13C. The movable contact element will then be moved in the opposite direction, perpendicularly away from the fixed element, which also deforms the sebaceous plug opposite direction (Figure 13D). This sequence is accomplished at a frequency within the range of 20-1,000 Hz, and preferably in the range of 80-200 Hz. Continued action dislodges or releases the sebaceous plug from the pore walls. The user slowly moves the device across the surface of the skin, producing the above results over an entire skin area.

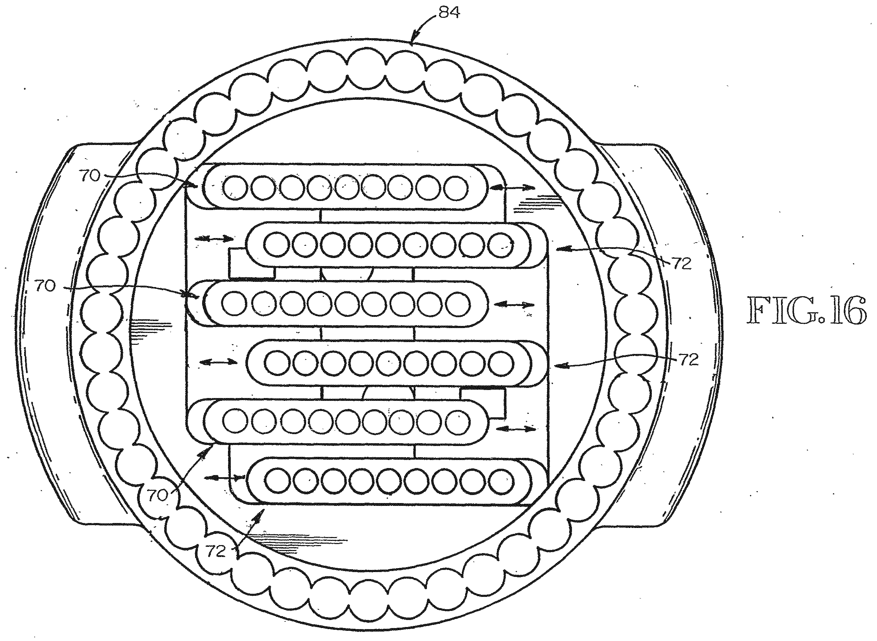

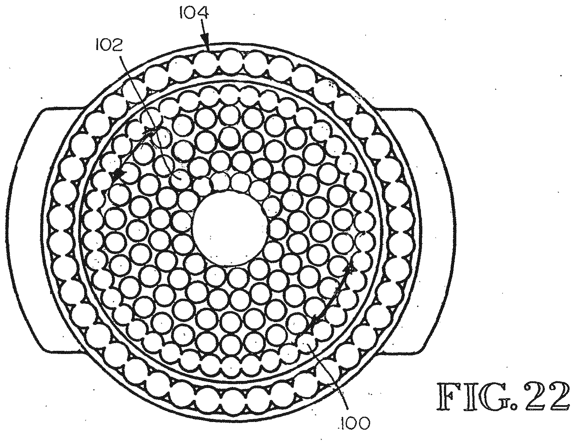

A further alternate mechanical configuration is shown in Figures 15-22. These configurations operate on substantially the same principles as the devices described above, but have contact elements composed of bristle tufts. In these embodiments, the base portions holding the bristle tufts are analogous to the mounting plates described above. Instead of rigid or compliant solid contact elements, a plurality of bristle tufts are employed. Each tuft is further composed of a plurality of filaments or bristles. The bristles may be made from any material suitable for the application, with the preferred material being Nylon 612. The diameter of each bristle is in the range of 50.8 µm (2 mils) to 127 µm (5 mils) with a preferred diameter of 76.2 µm (3 mils), and of lengths in the range 6.25 mm (0.25 inches) to 15.2 mm (0.60 inches), with a preferred length of 10.9 mm (0.43 inches) in length.

The base of the tuft has a diameter in the range of 1.0 mm (40 mils) to 2.54 mm (100 mils) with a preferred diameter of 1.5 mm (60 mils) for the tufts of the fixed and moving interior bristle tuft rows and a preferred diameter of 2.0 mm (80 mils) for the fixed exterior bristle tuft row. The diameter and length of the bristles determine their stiffness. Using the same material, larger diameter bristles are stiffer than smaller diameter bristles. Generally longer bristles are softer than shorter bristles. The material used to make the bristles also dictates the stiffness character of the bristles. Additionally, the rows can be made with individual tufts having a different number of bristles. Generally, having more bristles of a smaller diameter in a tuft will produce a softer sensation.

Tufts of 76.2 µm (0.003 inches) diameter Nylon 612 bristles 10.9 mm (0.43 inches) in length produce a lateral stiffness which works well in moving the skin within Region I and the lower part of Region II of Figure 4. Such tufts produce a lateral spring constant of approximately 3.94 grams/cm (10 grams/inch) at a displacement of 1.52 mm (0.06"); i.e. a lateral displacement of 1.52 mm (0.06") of the end of a tuft results when a lateral force of 0.6 grams is applied to the end of the tuft relative to the base.

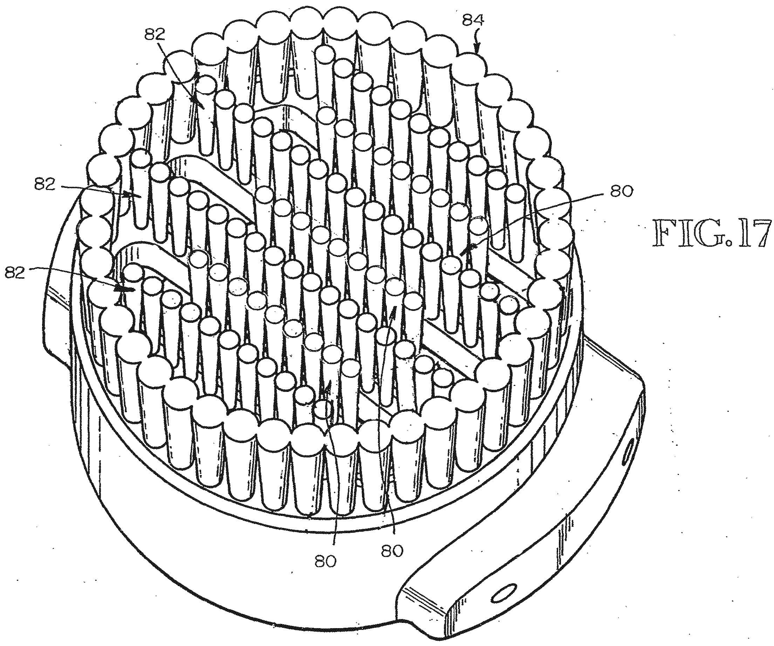

Figures 15-18 show an embodiment using linear motion of the bristle rows. In Figures 15-16, both sets of bristle rows (first set of three rows 70-70, second set of three rows 72-72) move with respect to each other, while in Figures 17-18, one set of four rows 82-82 is fixed and the other set of three rows 80-80 moves. In both embodiments, the rows of moving/fixed bristle tufts are surrounded by a circle of bristle tufts 84, the circle of bristles 84 being fixed and functioning like a curtain to keep cleanser/water on the skin.

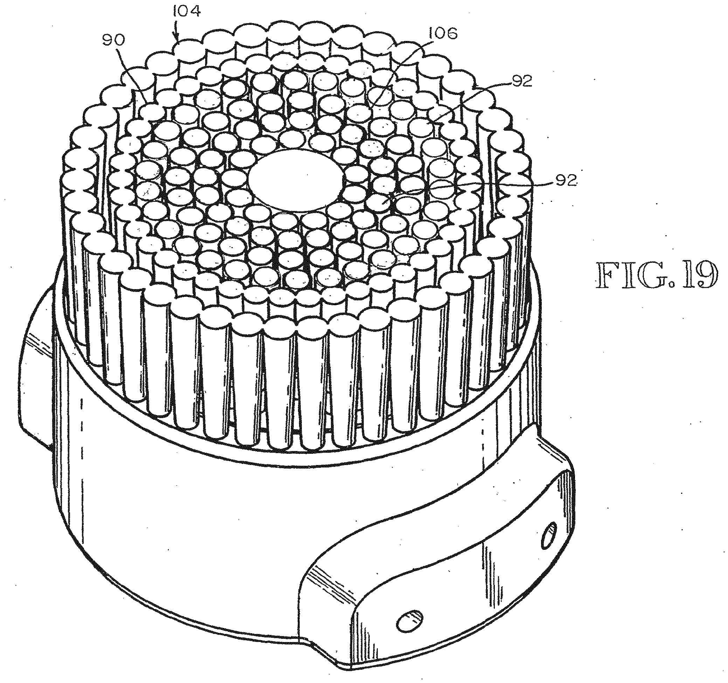

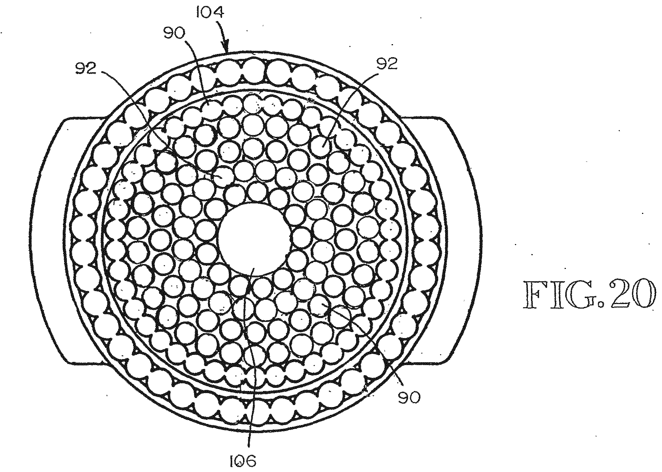

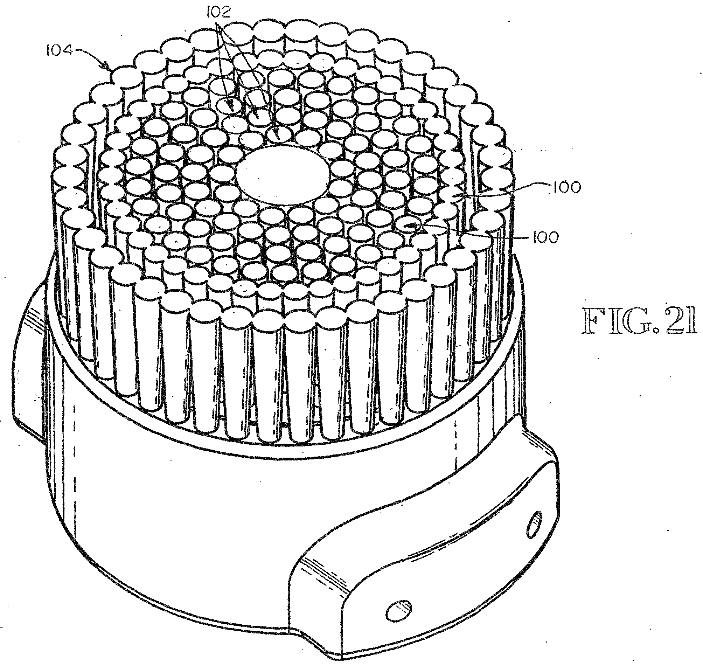

In another embodiment shown in Figures 19-22, the row(s) of bristle tufts are circular and move in an arcuate manner with the axis of rotation perpendicular to the surface of the skin. Figures 19-20 show an embodiment in which both sets of circular bristle rows (two rows 90-90 and two rows 92-92) move with respect to each other, while Figures 21-22 show an embodiment in which one set of two rows 100-100 moves and the other set of three rows 102-102 is fixed. In each case, one row each of 90 and 92 bristle rows and one row each of 100 and 102 bristle rows would likely be sufficient for cleansing action. Additional rows beyond that shown could also work. In both of these embodiments, the rows of bristles are encircled by a circle of fixed bristles 104, acting as a curtain for liquid, etc. In the embodiment of Figures 19 and 20, there is also a circle of fixed bristles 106 inside of the circle of rows 90 and 92.

The adjacent rows of bristle tufts for the devices shown in Figures 15-22 move relative to each other at an amplitude sufficient to deform the skin in region and slightly into region II of Figure 4 as shown to produce the cleansing action.

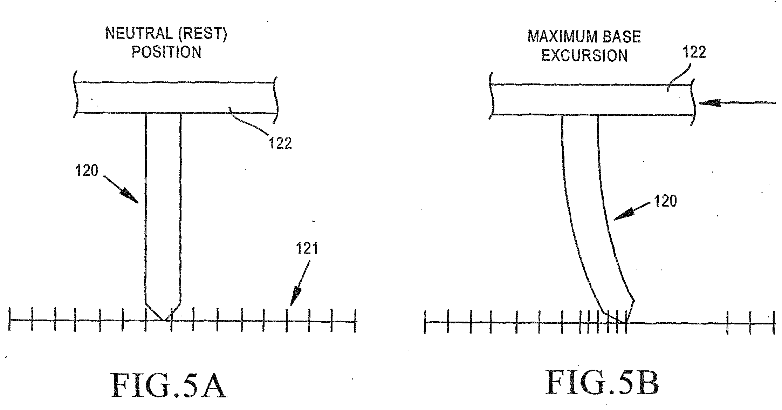

Figure 5A shows the cleansing action of bristles with a single tuft of bristles 120 against the skin 121 when base 122 is at rest (neutral), while Figure 5B shows the tuft 120 when the base 122 is at a maximum excursion. The skin 121 is indicated with "tick" marks on a horizontal line, with the tick spacing showing relative deformation of the skin.

In Figure 5A, the uniform spacing of the tick marks indicates that there is no deformation of the skin when the bristles are in the rest position. Figure 5B shows that the skin has been compressed slightly by the bristles in the direction of motion of the base, and stretched slightly behind the tips of the bristles. In typical operation, both the bristles and the skin deform, and there is relatively little slippage of the bristles on the skin. As the tuft base moves from its rest (neutral) position, the skin deformation increases, as does its modulus, until the restorative force of the skin just balances that of the bristles.

Typical peak-to-peak amplitudes measured at the base of the bristle tufts of 1.27 mm (0.05 inches) to 6.35 mm (0.25 inches) can be used with rows having center-to-center spacing of 2.54 mm (0.10 inches)-6.35 mm (0.25 inches). This results in the peak amplitude (50% of peak-to-peak amplitude) of typically 40%, and in a range of 10% to 100% of the center-to-center spacing between adjacent rows of tufts. At high amplitudes, the bristles may also slide across the surface, especially if the brush is used with lubricating elements.

Referring now again to Figures 19-22, lubricating fluid can be supplied by the device, for example, through a central port 106 shown in Figures 19 and 20. Centripetal force tends to spread the emitted fluid onto the bristles, supplying relatively uniform wetting. The fluid is contained by the bristle curtain 104.

In the case of rotational configurations such as shown in Figures 19-22, the linear amplitude of motion is larger for the outer rings. The center-to-center spacing can be adjusted among the rows to maintain an approximately constant ratio of amplitude to inter-element spacing.

The bristle rows described above can also be replaced with flexible members, such as an elastomer or closed cell foam.

It is also possible to combine the advantages of the differential shear mode and tension/compression modes described above into a compound motion, for example, elliptical.

It is also possible to apply bi-directional motion to the skin via a single set of contact elements for cleaning or clearing the infundibular opening. Unlike the cases above in which there is a differential reciprocating motion between adjacent contact elements, the use of a single set of elements relies on inertia of the skin to effect a differential force on the pore openings. The single set of moving contact elements, such as a row of bristles, forces the skin immediately adjacent to it to move. This movement is coupled to skin regions somewhat distant through the skin's elasticity. However, skin also has inertia which resists motion, thereby producing a shear force in the direction of movement. This shear force decreases at greater distances from the moving contact elements.

Applying bi-directional reciprocating movement via a single set of contact elements is generally not as effective as using adjacent contact elements arranged to apply tension/compression or shear between them.

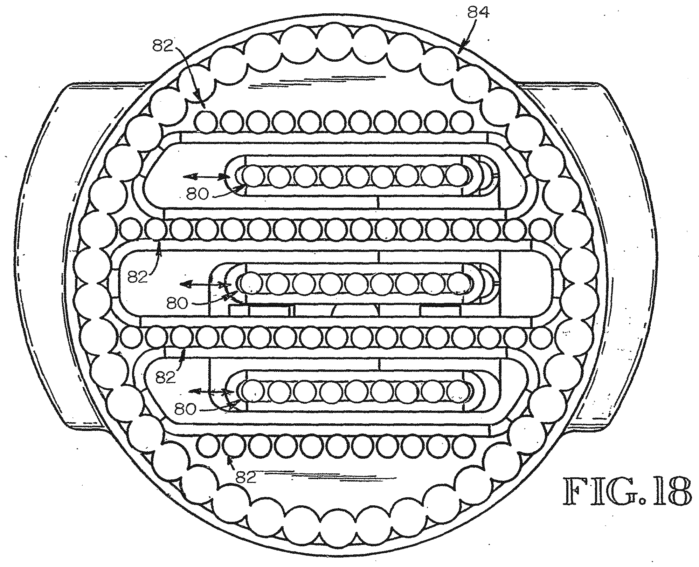

The single set of reciprocating contact elements can be implemented in a linear manner, such as the device of Figures 17 and 18, with all linear bristles moving in unison. This single set reciprocating motion can also be implemented in an arcuate manner, such as the device of Figures 21-22, with all rows of bristles moving in unison. Where the contacting element comprises rigid or compliant material, the peak-to-peak amplitude of movement will be within the range of 1.02 mm (0.04 inches)-3.81 mm (0.150 inches), preferably 2.29 mm (0.09 inches); if the contacting element is a row of bristles, the peak-to-peak amplitude of movement is within the range of 0.508 mm (0.020 inches) to 4.06 mm (0.160 inches), preferably 2.08 mm (0.082 inches).

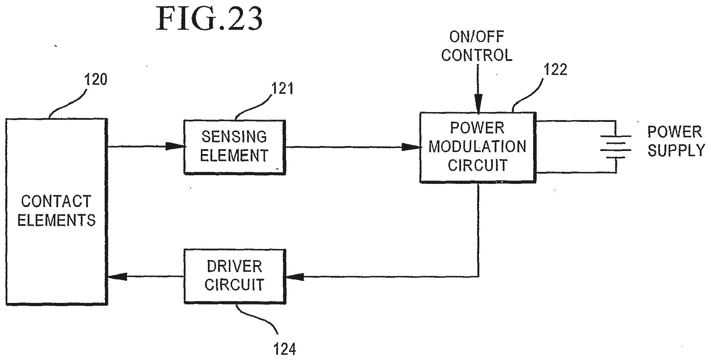

Figure 23 shows a schematic block diagram for a control means for controlling the amplitude of the moving portions of the contact elements 120. The control means is composed of a sensing element 121, a power modulation circuit 122, and a driver circuit 124. The sensing element 121 senses the amount of pressure applied to the skin by the contact elements 120 and applies a signal to the power modulation circuit 122. The power modulation circuit 122 uses said signal to modulate the power of the driver circuit 124 and amplitude of the contact elements 120.

The control means operates in a plurality of operating modes with a preferred number of three operating modes. Proper operation of the apparatus requires that the pressure applied to the skin by the applicator remains in given range.

When pressure applied by the contact elements to the skin is below the lower threshold for proper operation of the apparatus, the amplitude of the applicator with the contact elements is substantially reduced from its nominal amplitude. This reduces the likelihood of splashing of fluids or cleaning agents when the applicator is not in contact with the skin.

When pressure applied by the applicator to the skin is above the lower threshold for proper operation of the apparatus, but below the upper threshold, the applicator is driven at nominal amplitude.

When pressure applied to the skin by the applicator is above the upper threshold for proper operation the amplitude of the applicator with the contact elements is substantially reduced from its normal amplitude or, preferably, stopped altogether. An alternative is to interrupt the power to the contact elements at a low frequency, for example 2-10Hz, in order to create an audible or tactile feedback to the user to reduce the pressure. This excess pressure feedback signal reduces the likelihood that the applicator will cause too much motion of the skin.

The above-described control means provides not only safety and convenience, but also provides feedback to the user to maintain applicator pressure in the range for proper operation of the apparatus.

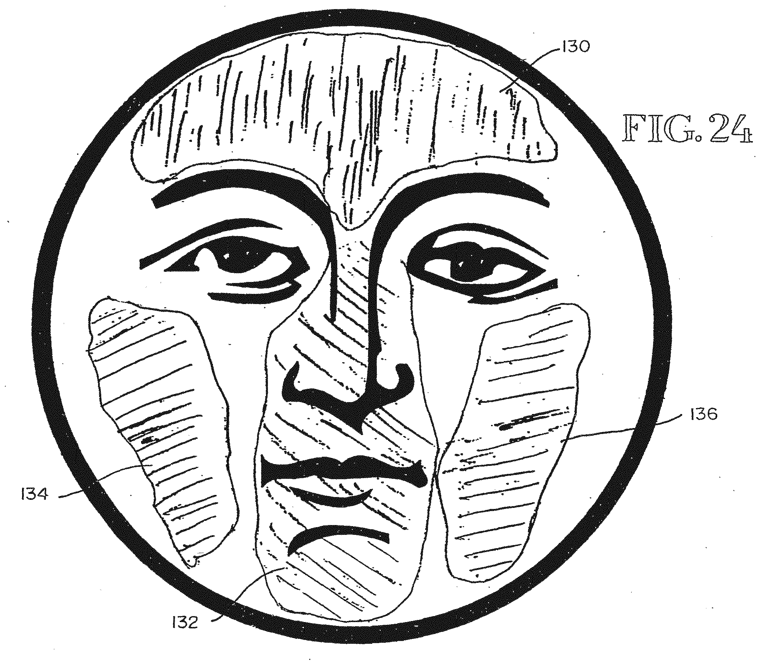

Figure 24 shows four regions of the face that differ in topology and degree of sebaceous secretion. Generally the face is divided into two distinct regions, the so-called "T-zone" 130 and 132, and the outer cheek areas 134, 136. The T-zone is the part of the face consisting of the forehead, nose and the area around the mouth, including the chin. It is so named because it is shaped like the letter T.

Often the T-zone is more prone to acne, as the percentage of sebum glands in this area tends to be higher than on the outer cheeks. An important component of the present invention, therefore, is a timer means to assist the user in properly treating the differing zones of the face, according to the typical incidence of acne in that area, without over- or under-treating the area. The total treatment time may be from 30 seconds to two minutes and preferably one minute. Further, the total time may be subdivided into two or more and preferably four time periods. In the present invention, the first time period is 20 seconds for treatment of the forehead 130; the second time period is 20 seconds for treatment of the nose, perioral area and chin 132; and 10 seconds for each outer cheek areas 134, 136.

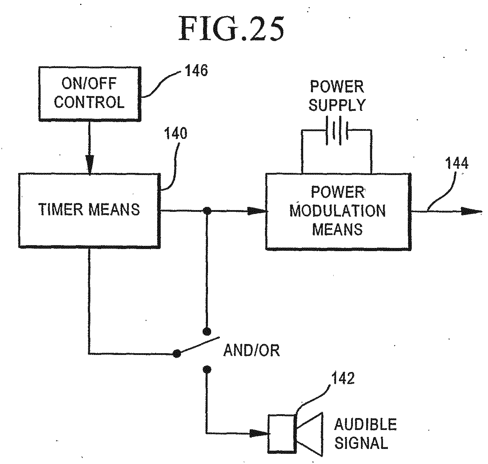

A timer means 140 (Figure 25) prompts the user by providing either an audible signal 142 or a detectable change in the motion of the moving contacting element, or both simultaneously. This prompt instructs the user when the preferred treatment time has elapsed for each area of the face. The timer can be enabled or disabled after the apparatus is turned on, by means of an on/off switch 146 being pressed on for a selected period of time. A first audible signal can be used to indicate that the timer has been enabled and a second audible signal to indicate that the timer has been disabled.

In summary, applying differential motion locally to the infundibular (pore) opening results in the clearing of sebaceous plugs from the acroinfundibulum (top of the pore). The differential motion, whether linear, arcuate or elliptical, applies forces to the interface between the comedone (sebaceous plug) and the surrounding tissue, thus breaking the adhesion between the acroinfundibular wall and the sebaceous plug.

A bi-directional, return-to-center motion generally provides better cleaning than unidirectional motion due to the nature of the sebaceous plug, i.e. the sebaceous plug can be thought of as a generally disorganized matrix of flat, brick-like corneocytes embedded in a "mortar" of oxidized sebum lipids. Adhesion of the plug to the wall of the acroinfundibulum is thought to be caused by a combination of oxidized sebum and ceramide lipids. Because the orientation of the corneocytes is not completely random relative to the wall of the acroinfundibulum, it is possible that unidirectional motion alone would eliminate some of the adhesion but may be insufficient to loosen the sebum plug. The preferred embodiment of the invention applies bi-directional motion such that most or all of the corneocytes are subject to adhesion-breaking stresses irrespective of their orientation.

Limiting the amplitude of bi-directional motion to an extent which generally maintains the skin in a region of low strain is also beneficial. High amplitude bi-directional or unidirectional motion places the collagen fibers in a higher strain condition.

In use, our invention applies cyclic deformation and relaxation many times per second to the skin surrounding the acroinfundibulum and any sebaceous plug. The repetition of differential vibratory cycles supplies a therapeutic effect by gradually breaking the adhesion between the acroinfundibulum and the sebaceous plug.

The present invention is intended to operate in a frequency range of 20-1,000 Hz. A preferred range is 80-200 Hz. Below 80 Hz, the vibration rate is less than optimal and the mechanical implementation is more difficult. Above 200 Hz, a strong tickle reaction, usually unpleasant, occurs in the nose region. Assuming a 1 cm width of active surface of the device operating at the minimum frequency, moving the device across the skin surface linearly at 2 cm/sec would result in each pore experiencing 10 deformation cycles, many more times than would be practicable by any manual technique. At higher frequencies the number of deformations per stroke of the appliance would be proportionally greater.

There are two basic modes of differential movement that can be applied: shear and tension/compression. The shear mode device applies a linear differential motion via narrow elements which contact the skin, and which move in the direction of their length with respect to each other. The device typically applies a sinusoidal oscillation to adjacent contact elements. The arrangement includes two contact element assemblies. The device moves the contact elements in parallel to each other along their long axis. Sufficient frictional forces between the surface of the contact elements and the skin surface will transfer this motion to the skin, creating a shear action on the skin between them as shown in Figures 9A-9D.

The tension/compression mode device, in contrast to shear mode, moves the contact elements toward and away from each other. The oscillations are perpendicular to the long axis of the contact elements (i.e. one element moving toward one neighbor and away from its other neighbor), thus creating alternating tension and compression stress in the tissue surrounding the infundibulum. Sufficient frictional forces between the surface of the contact elements and the skin surface will transfer this motion to the skin as shown in Figures 13A-13D.

Alternatively to one contact element moving, both contact elements may move with respect to the device body, and counter to one another.

The skin contacting elements can be rigid or flexible. Rigid surfaces can be made from stainless steel and plastic. Flexible contacting surfaces can include bristles, elastomers and soft compliant foam. The surfaces should have sufficient roughness in order to transfer the motion to the skin without slippage, or minimizing such slippage. Additionally, the proper degree of surface roughness assures good lamellar action (transfer of lubricant from wet to dry portion of skin by interstitial spaces in the contacting surface). If the surface finish is too smooth the lubrication is wiped off and the contacting surface runs dry against skin and may cause unwanted abrasion of the skin. This surface roughness can be in the range of 127 µm (5 micro-inches) to 508 µm (20 micro-inches) and preferably is 254 µm (10 micro-inches).

Multiple contacting elements can be included, such that a set of skin contacting elements moving in one direction are interdigitated between a set of stationary skin contacting elements, or skin contacting elements moving in the opposing direction. Figure 15 shows a device with a double pair of skin contacting elements, and is a derivative of the device with a single pair of skin contacting elements shown in Figure 6. In the case shown in Figure 15, each of two sets of skin contacting elements consists of three rows of bristle tufts. Each set of bristle tuft rows moves in relative opposition, surrounded by a fixed circular row of fixed bristle tufts, which serve to minimize splashing and to control the contact of the moving bristle tips onto the skin. The bristle tufts are designed so that the interdigitated row movement results in sufficient force on the skin to maintain acroinfundibular openings. This action is similar to fingers of left and right hands interlocking while hand washing.

The model shown has three rows of bristles in each of the two interdigitated sets, but the number of bristle rows could vary from a single row to as many as practical for the desired surface area.

Additionally, the motion of the bristle tuft row(s) can be linear, arcuate or elliptical along the plane of the skin with the axis of rotation perpendicular to the skin.

The magnitude of reciprocal force applied to the skin is primarily determined by the stiffness of bristle tufts to lateral deformation, the length and width of the bristle rows, spacing between bristle rows, amplitude of interdigitated motion, and the pressure applied by the user.

The effects on the skin by the movement of the contacting elements can also be modified with the use of a skin lubricant. The lubricant can be water, soapy water, another skin cleaning agent, a lotion or gel. More lubrication results in.more sliding action of the bristle tips across the skin, and less deforming action applied to the skin. The sliding action across the skin serves to remove skin surface debris. The debris includes sebum, triglycerides and fatty acids, desquamatized corneocytes and accumulated dirt and environmental materials.

Thus, the present invention provides either mechanical energy in a shear mode or tension/compression mode or a combination (elliptical) in order to loosen the adhesion between the sebaceous plug and the walls of the pore. Said motion can be produced by contact elements moving either reciprocally linearly, reciprocally arcuately or a combination thereof. The loosened sebaceous plug and any previously blocked lipids from the pores can then be readily removed by rinsing the cleansed area. Such an arrangement results in an effective treatment of early stage acne that prevents the development of more serious acne conditions. In addition, however, the arrangement can be used for effective cleansing of skin when acne is not present. The combination of gentleness and cleansing action produces a desirable, effective cleansing effect on the skin and a "sense" or feel by the user of clean, healthy skin.

The control means operates in a plurality of operating modes with a preferred number of three operating modes. Proper operation of the apparatus requires that the pressure applied to the skin by the applicator remains in given range.

When pressure applied by the contact elements to the skin is below the lower threshold for proper operation of the apparatus, the amplitude of the applicator with the contact elements is substantially reduced from its nominal amplitude. This reduces the likelihood of splashing of fluids or cleaning agents when the applicator is not in contact with the skin.

When pressure applied by the applicator to the skin is above the lower threshold for proper operation of the apparatus, but below the upper threshold, the applicator is driven at nominal amplitude.

When pressure applied to the skin by the applicator is above the upper threshold for proper operation the amplitude of the applicator with the contact elements is substantially reduced from its normal amplitude or, preferably, stopped altogether. An alternative is to interrupt the power to the contact elements at a low frequency, for example 2-10Hz, in order to create an audible or tactile feedback to the user to reduce the pressure. This excess pressure feedback signal reduces the likelihood that the applicator will cause too much motion of the skin.

The above-described control means provides not only safety and convenience, but also provides feedback to the user to maintain applicator pressure in the range for proper operation of the apparatus.

Figure 24 shows four regions of the face that differ in topology and degree of sebaceous secretion. Generally the face is divided into two distinct regions, the so-called "T-zone" 130 and 132, and the outer cheek areas 134, 136. The T-zone is the part of the face consisting of the forehead, nose and the area around the mouth, including the chin. It is so named because it is shaped like the letter T.

Often the T-zone is more prone to acne, as the percentage of sebum glands in this area tends to be higher than on the outer cheeks. An important component of the present invention, therefore, is a timer means to assist the user in properly treating the differing zones of the face, according to the typical incidence of acne in that area, without over- or under-treating the area. The total treatment time may be from 30 seconds to two minutes and preferably one minute. Further, the total time may be subdivided into two or more and preferably four time periods. In the present invention, the first time period is 20 seconds for treatment of the forehead 130; the second time period is 20 seconds for treatment of the nose, perioral area and chin 132; and 10 seconds for each outer cheek areas 134, 136.

A timer means 140 (Figure 25) prompts the user by providing either an audible signal 142 or a detectable change in the motion of the moving contacting element, or both simultaneously. This prompt instructs the user when the preferred treatment time has elapsed for each area of the face. The timer can be enabled or disabled after the apparatus is turned on, by means of an on/off switch 146 being pressed on for a selected period of time. A first audible signal can be used to indicate that the timer has been enabled and a second audible signal to indicate that the timer has been disabled.

In summary, applying differential motion locally to the infundibular (pore) opening results in the clearing of sebaceous plugs from the acroinfundibulum (top of the pore). The differential motion, whether linear, arcuate or elliptical, applies forces to the interface between the comedone (sebaceous plug) and the surrounding tissue, thus breaking the adhesion between the acroinfundibular wall and the sebaceous plug.

A bi-directional, return-to-center motion generally provides better cleaning than unidirectional motion due to the nature of the sebaceous plug, i.e. the sebaceous plug can be thought of as a generally disorganized matrix of flat, brick-like corneocytes embedded in a "mortar" of oxidized sebum lipids. Adhesion of the plug to the wall of the acroinfundibulum is thought to be caused by a combination of oxidized sebum and ceramide lipids. Because the orientation of the corneocytes is not completely random relative to the wall of the acroinfundibulum, it is possible that unidirectional motion alone would eliminate some of the adhesion but may be insufficient to loosen the sebum plug. The preferred embodiment of the invention applies bi-directional motion such that most or all of the corneocytes are subject to adhesion-breaking stresses irrespective of their orientation.

Limiting the amplitude of bi-directional motion to an extent which generally maintains the skin in a region of low strain is also beneficial. High amplitude bi-directional or unidirectional motion places the collagen fibers in a higher strain condition.

In use, our invention applies cyclic deformation and relaxation many times per second to the skin surrounding the acroinfundibulum and any sebaceous plug. The repetition of differential vibratory cycles supplies a therapeutic effect by gradually breaking the adhesion between the acroinfundibulum and the sebaceous plug.

The present invention is intended to operate in a frequency range of 20-1,000 Hz. A preferred range is 80-200 Hz. Below 80 Hz, the vibration rate is less than optimal and the mechanical implementation is more difficult. Above 200 Hz, a strong tickle reaction, usually unpleasant, occurs in the nose region. Assuming a 1 cm width of active surface of the device operating at the minimum frequency, moving the device across the skin surface linearly at 2 cm/sec would result in each pore experiencing 10 deformation cycles, many more times than would be practicable by any manual technique. At higher frequencies the number of deformations per stroke of the appliance would be proportionally greater.

There are two basic modes of differential movement that can be applied: shear and tension/compression. The shear mode device applies a linear differential motion via narrow elements which contact the skin, and which move in the direction of their length with respect to each other. The device typically applies a sinusoidal oscillation to adjacent contact elements. The arrangement includes two contact element assemblies. The device moves the contact elements in parallel to each other along their long axis. Sufficient frictional forces between the surface of the contact elements and the skin surface will transfer this motion to the skin, creating a shear action on the skin between them as shown in Figures 9A-9D.

The tension/compression mode device, in contrast to shear mode, moves the contact elements toward and away from each other. The oscillations are perpendicular to the long axis of the contact elements (i.e. one element moving toward one neighbor and away from its other neighbor), thus creating alternating tension and compression stress in the tissue surrounding the infundibulum. Sufficient frictional forces between the surface of the contact elements and the skin surface will transfer this motion to the skin as shown in Figures 13A-13D.

Alternatively to one contact element moving, both contact elements may move with respect to the device body, and counter to one another.

The skin contacting elements can be rigid or flexible. Rigid surfaces can be made from stainless steel and plastic. Flexible contacting surfaces can include bristles, elastomers and soft compliant foam. The surfaces should have sufficient roughness in order to transfer the motion to the skin without slippage, or minimizing such slippage. Additionally, the proper degree of surface roughness assures good lamellar action (transfer of lubricant from wet to dry portion of skin by interstitial spaces in the contacting surface). If the surface finish is too smooth the lubrication is wiped off and the contacting surface runs dry against skin and may cause unwanted abrasion of the skin. This surface roughness can be in the range of 5 to 20 micro-inches and preferably is 10 micro-inches (0.013 to 0.051cm and preferably is 0.025cm).

Multiple contacting elements can be included, such that a set of skin contacting elements moving in one direction are interdigitated between a set of stationary skin contacting elements, or skin contacting elements moving in the opposing direction. Figure 15 shows a device with a double pair of skin contacting elements, and is a derivative of the device with a single pair of skin contacting elements shown in Figure 6. In the case shown in Figure 15, each of two sets of skin contacting elements consists of three rows of bristle tufts. Each set of bristle tuft rows moves in relative opposition, surrounded by a fixed circular row of fixed bristle tufts, which serve to minimize splashing and to control the contact of the moving bristle tips onto the skin. The bristle tufts are designed so that the interdigitated row movement results in sufficient force on the skin to maintain acroinfundibular openings. This action is similar to fingers of left and right hands interlocking while hand washing.

The model shown has three rows of bristles in each of the two interdigitated sets, but the number of bristle rows could vary from a single row to as many as practical for the desired surface area.

Additionally, the motion of the bristle tuft row(s) can be linear, arcuate or elliptical along the plane of the skin with the axis of rotation perpendicular to the skin.

The magnitude of reciprocal force applied to the skin is primarily determined by the stiffness of bristle tufts to lateral deformation, the length and width of the bristle rows, spacing between bristle rows, amplitude of interdigitated motion, and the pressure applied by the user.

The effects on the skin by the movement of the contacting elements can also be modified with the use of a skin lubricant. The lubricant can be water, soapy water, another skin cleaning agent, a lotion or gel. More lubrication results in.more sliding action of the bristle tips across the skin, and less deforming action applied to the skin. The sliding action across the skin serves to remove skin surface debris. The debris includes sebum, triglycerides and fatty acids, desquamatized corneocytes and accumulated dirt and environmental materials.

Thus, the present invention provides either mechanical energy in a shear mode or tension/compression mode or a combination (elliptical) in order to loosen the adhesion between the sebaceous plug and the walls of the pore. Said motion can be produced by contact elements moving either reciprocally linearly, reciprocally arcuately or a combination thereof. The loosened sebaceous plug and any previously blocked lipids from the pores can then be readily removed by rinsing the cleansed area. Such an arrangement results in an effective treatment of early stage acne that prevents the development of more serious acne conditions. In addition, however, the arrangement can be used for effective cleansing of skin when acne is not present. The combination of gentleness and cleansing action produces a desirable, effective cleansing effect on the skin and a "sense" or feel by the user of clean, healthy skin.

## Claims

1. An apparatus for treatment of skin, comprising:
- at least one oscillating contacting element (57, 59) comprising a row of bristle tufts (84) having an end face for contacting the skin of a user; and
- an assembly (50) for oscillating said contacting element back and forth in a bi-directional manner, such that when the apparatus is positioned so that the contacting element end face contacts the skin, an action on the skin is produced to remove undesired material from the skin pores, wherein the frequency of oscillation of the contacting element is within a range of 80-200 Hz, and wherein the bristles have a diameter in the range of 0,0508 mm (2 mils) to 0,127 mm (5 mils) and a length in the range of 6,35 mm (0.250 inches) to 15,24 mm (0.600 inches), the motion of oscillating of said contacting element being linear, arcuate or elliptical along the plane of the skin when the apparatus is positioned so that the contacting element end face contacts the skin.

2. The apparatus of claim 1, wherein oscillation of the contacting element places the skin in alternating shear, which assists in loosening the sebaceous plugs from the skin pores in which they are located.

3. The apparatus of claim 1, wherein the oscillation of the contacting element reciprocally places the skin in alternating tension and compression.

4. The apparatus of claim 1, wherein the bristles have a diameter of 0,0762 mm (3 mils).

5. The apparatus of claim 1, wherein the bristles have length of 10,9222 mm (0,43 inches).

6. The apparatus of claim 1, wherein the bristles material has a flexural modulus in the range of 689 to 6894 MPa (100 to 1000 kpsi).

7. The apparatus of claim 6, wherein the bristles material has a flexural modulus of 4136 MPa (600 kpsi).

8. The apparatus of claim 1, wherein the peak-to-peak amplitude of the motion of the bristle tufts is within the range of 0.508 mm to 4,064 mm (0.020 to 0.160 inches).

9. The apparatus of claim 1, wherein the row of bristle tufts is arranged in a straight line.

10. The apparatus of claim 1, wherein the row of bristle tufts is arranged in a circle.

11. The apparatus of claim 1, comprising:
at least two contacting elements having end faces, in the same plane, wherein at least one contacting element is a oscillating contacting element;
a mounting assembly (32) for holding the contacting elements adjacent to each other; and
an assembly for reciprocally oscillating said at least one oscillating contacting element relative to at least one adjacent contacting element.

## Patentansprüche

1. Vorrichtung für die Behandlung der Haut, die Folgendes umfasst:
- wenigstens ein hin und her bewegliches Kontaktierungselement (57, 59), das eine Reihe von Borstenbündeln (84) enthält, die eine Stirnfläche für die Herstellung eines Kontakts mit der Haut eines Benutzers besitzen; und
- eine Anordnung (50) zum Hin- und Herbewegen des Kontaktierungselements auf bidirektionale Weise rückwärts und vorwärts, derart, dass dann, wenn die Vorrichtung so positioniert ist, dass die Stirnfläche des Kontaktierungselements mit der Haut in Kontakt ist, eine Einwirkung auf die Haut hervorgerufen wird, um unerwünschtes Material aus den Hautporen zu entfernen, wobei die Frequenz der Hin- und Herbewegung des Kontaktierungselements in einem Bereich von 80-200 Hz liegt und wobei die Borsten einen Durchmesser im Bereich von 0,0508 mm (2 mils) bis 0,127 mm (5 mils) und eine Länge im Bereich von 6,35 mm (0,250 Zoll) bis 15,24 mm (0,600 Zoll) besitzen, wobei die Bewegung der Hin- und Herbewegung des Kontaktierungselements in der Ebene der Haut geradlinig, gekrümmt oder elliptisch ist, wenn die Vorrichtung so positioniert ist, dass die Stirnfläche des Kontaktierungselements mit der Haut in Kontakt ist.

2. Vorrichtung nach Anspruch 1, wobei die Hin- und Herbewegung des Kontaktierungselements die Haut in eine abwechselnde Scherung versetzt, was die Loslösung der Talgpfropfen aus den Hautporen, in denen sie sich befinden, unterstützt.

3. Vorrichtung nach Anspruch 1, wobei die Hin- und Herbewegung des Kontaktierungselements die Haut abwechselnd auseinanderzieht und komprimiert.

4. Vorrichtung nach Anspruch 1, wobei die Borsten einen Durchmesser von 0,0762 Millimeter (3 mils) haben.

5. Vorrichtung nach Anspruch 1, wobei die Borsten eine Länge von 10,9222 mm (0,43 Zoll) haben.

6. Vorrichtung nach Anspruch 1, wobei das Borstenmaterial einen Biegemodul im Bereich von 689 bis 6894 MPa (100 bis 1000 kpsi) hat.

7. Vorrichtung nach Anspruch 6, wobei das Borstenmaterial einen Biegemodul von 4136 MPa (600 kpsi) hat.

8. Vorrichtung nach Anspruch 1, wobei die Spitze/Spitze-Amplitude der Bewegung der Borstenbündel im Bereich von 0,508 Millimeter bis 4,064 Millimeter (0,020 bis 0,160 Zoll) liegt.

9. Vorrichtung nach Anspruch 1, wobei die Reihe von Borstenbündeln in einer geraden Linie angeordnet ist.

10. Vorrichtung nach Anspruch 1, wobei die Reihe von Borstenbündeln in einem Kreis angeordnet ist.

11. Vorrichtung nach Anspruch 1, die Folgendes umfasst:
wenigstens zwei Kontaktierungselemente, die Stirnflächen in derselben Ebene besitzen, wobei wenigstens ein Kontaktierungselement ein hin und her bewegliches Kontaktierungselement ist;
eine Befestigungsanordnung (32) zum Halten der Kontaktierungselemente benachbart zueinander; und
eine Anordnung zum Hin- und Herbewegen des wenigstens einen hin und her beweglichen Kontaktierungselements relativ zu wenigstens einem benachbarten Kontaktierungselement.

## Revendications

1. Appareil pour le traitement de la peau comprenant :
au moins un élément de contact oscillant (57, 59) comprenant une rangée de touffes de poils (84) ayant une face d'extrémité pour entrer en contact avec la peau d'un utilisateur ; et
un ensemble (50) pour faire osciller ledit élément de contact vers l'avant et vers l'arrière d'une manière bidirectionnelle, de sorte que lorsque l'appareil est positionné de sorte que la face d'extrémité de l'élément de contact est en contact avec la peau, une action sur la peau est produite pour retirer la matière indésirée des pores de la peau, dans lequel la fréquence d'oscillation de l'élément de contact est dans une plage de 80 à 200 Hz, et dans lequel les poils ont un diamètre dans la plage de 0,0508 mm (2 mils) à 0,127 mm (5 mils) et une longueur dans la plage de 6,35 mm (0,250 pouce) à 15,24 mm (0,600 pouce), le mouvement oscillatoire dudit élément de contact étant linéaire, arqué ou elliptique le long du plan de la peau lorsque l'appareil est positionné de sorte que la face d'extrémité de l'élément de contact est en contact avec la peau.

2. Appareil selon la revendication 1, dans lequel l'oscillation de l'élément de contact place la peau en cisaillement alternatif, ce qui aide à se débarrasser des bouchons sébacés des pores de la peau dans lesquels ils sont positionnés.

3. Appareil selon la revendication 1, dans lequel l'oscillation de l'élément de contact place, de manière réciproque, la peau en tension et en compression alternées.

4. Appareil selon la revendication 1, dans lequel les poils ont un diamètre de 0,0762 mm (3 mils).

5. Appareil selon la revendication 1, dans lequel les poils ont une longueur de 10,9222 mm (0,43 pouce).

6. Appareil selon la revendication 1, dans lequel le matériau des poils a un module de flexion dans la plage de 689 à 6894 MPa (100 à 1000 kpsi).

7. Appareil selon la revendication 1, dans lequel le matériau des poils a un module de flexion de 4136 MPa (600 kpsi).

8. Appareil selon la revendication 1, dans lequel l'amplitude crête à crête du mouvement des touffes de poils est dans la plage de 0,508 mm à 4,064 mm (0,020 à 0,160 pouce).

9. Appareil selon la revendication 1, dans lequel la rangée de touffes de poils est agencée sur une ligne droite.

10. Appareil selon la revendication 1, dans lequel la rangée de touffes de poils est agencée sur un cercle.

11. Appareil selon la revendication 1, comprenant :
au moins deux éléments de contact ayant des faces d'extrémité, dans le même plan, dans lequel au moins un élément de contact est un élément de contact oscillant ;
un ensemble de montage (32) pour maintenir les éléments de contact adjacents entre eux ; et
un ensemble pour faire osciller, de manière réciproque, ledit au moins un élément de contact oscillant par rapport à au moins un élément de contact adjacent.
